# EUROPEAN PATENT SPECIFICATION

(11) **EP 3 527 170 B1**
(45) Date of publication and mention of the grant of the patent: **18.11.2020**
(21) Application number: 18157633.1
(22) Date of filing: 20.02.2018
(51) Int. Cl.: A61F 2/24

(54) **DEVICE FOR ASSISTING A PRACTITIONER IN ADJUSTING THE LENGTH OF AN ARTIFICIAL CHORDAE IMPLANTED IN AN ATRIO-VENTRICULAR HEART VALVE**
VORRICHTUNG ZUR UNTERSTÜTZUNG EINES ARZTES BEI DER ANPASSUNG DER LÄNGE EINES KÜNSTLICHEN CHORDAE, DER IN EINER ATRIOVENTRIKULÄREN HERZKLAPPE IMPLANTIERT IST
DISPOSITIF POUR AIDER UN PRATICIEN DANS LE RÉGLAGE DE LA LONGUEUR D'UN CORDAGE ARTIFICIEL IMPLANTÉ DANS UNE VALVULE CARDIAQUE AURICULO-VENTRICULAIRE

(43) Date of publication of application: 21.08.2019
(73) Proprietor: Institut National des Sciences Appliquées de Lyon, 69100 Villeurbanne (FR); UNIVERSITE CLAUDE BERNARD - LYON 1, 69100 Villeurbanne (FR); Hospices Civils de Lyon, 69002 Lyon (FR)
(72) Inventor: OBADIA, Jean-François, 69003 LYON (FR); GRINBERG, Daniel, NEW YORK CITY, New York 10029 (US); COTTINET, Pierre-Jean, 69480 POMMIERS (FR); COTTINET, Minh-Quyen, 69480 POMMIERS (FR); CAPSAL, Jean-Fabien, 69350 LA MULATIERE (FR); AUDIGIER, David, 69300 CALUIRE ET CUIRE (FR)
(74) Representative: Be IP

(56) References cited:
- WO-A2-2015/138306
- US-A1- 2011 288 635

## Description

### FIELD OF THE INVENTION

The present invention relates to device for assisting a practitioner in attaching artificial chords (and more precisely *"artificial chordae tendineae",* called *"chordae"* hereafter) to a flailing or prolapsing leaflet in a beating heart.

More particularly, the present invention relates to a device for assessing and adjusting the tension of artificial chordae (hereafter called *"neochordae"*) during transapical neochordae implantation.

### BACKGROUND OF THE INVENTION

Various types of surgical procedures are currently performed to treat diseases of the heart.

Some of these procedures concerns repair of mitral, aortic, and/or other heart valves.

The mitral valve inside the human heart include an annulus, two leaflets and a subvalvular apparatus. The subvalvular apparatus includes multiple chordae tendineae, which connect the mobile valve leaflets to muscular structures (papillary muscles) inside the ventricles. Rupture or elongation of the chordae tendineae result in partial or generalized leaflet prolapse, which causes mitral (or tricuspid) valve regurgitation.

A commonly used technique to surgically correct mitral valve regurgitation is the implantation of artificial chordae (i.e. neochordae) (usually 4-0 or 5-0 Gore-Tex sutures) between the prolapsing segment of the valve and the papillary muscle.

Indeed, the adjustment of the tension of each neochordae is difficult and approximate since it is performed manually: if the fixed neochordae is (are) too long or too short, then the coaptation of the leaflet is not optimal so that mitral regurgitation occurs.

WO2015/138306 **discloses an apparatus for positioning an epicardial anchor device and measuring the load of a tether extending from a prosthetic heart valve and coupled to the epicardial anchor device. The apparatus can include a handle assembly coupled to an elongate member and a docking member coupled to a distal end of the elongate member. The docking member can be releasably coupled to an epicardial anchor device configured to secure a tether extending from a prosthetic heart valve implanted with a heart at a location on an exterior of a ventricular wall of the heart. A force sensor device is coupled to the handle assembly and can measure a force exerted on the force sensor device. The force is associated with a tension of the tether extending through the elongate member and handle assembly.**

An aim of the present invention is to provide a device allowing a more precise and reproducible adjustment of the neochordae length during the treatment mitral valve disease.

### SUMMARY OF THE INVENTION

For this purpose, the invention proposes a device for assisting a practitioner in adjusting the length of artificial chordae implanted in an heart valve, the device comprising a plurality of gripping elements, each gripping element handling an end of a respective artificial chordae extending outward from the patient so that the device remains outside from the patient, *wherein* the device further comprises a plurality of force sensors, each force sensor being fixed to a respective gripping element for measuring a signal representative of a tension of the chordae **held** by the gripping element associated to the force sensor.

Preferred - but non-limiting - aspects of the device according to the invention are the following:
- the device further comprises a plate including a plurality of secondary translational motion elements, each secondary translational motion element supporting a respective force sensor so that each force sensor is slidably mounted on the plate;
- each secondary translational motion element allows individually moving a respective force sensor in translation, each secondary translational motion element including:
   ∘ a secondary endless screw assembly on which a respective force sensor is fixed,
   ∘ a secondary setting wheel fixed on one end of the secondary endless screw assembly extending longitudinally outward from the plate;
- each secondary endless screw assembly has a linear travel of 100 millimeters and a pitch distance per revolution of 1 millimeter;
- the device further comprises a base including a primary translational motion element connected to the plurality of force sensors so that each force sensor is slidably mounted on the base;
- the primary translational motion element allows synchronously moving the plurality of force sensors in translation, the primary translational motion element including:
   ∘ a primary endless screw assembly connected to the plurality of force sensors,
   ∘ a primary setting wheel fixed on one end of the primary endless screw assembly extending longitudinally outward from the base;
- the primary endless screw assembly has a linear travel of 100 millimeters and a pitch distance per revolution of 1 millimeter;
- the plate is fixed on the primary translational motion element so that the plate is slidably mounted on the base;
- each gripping element consists in a spring clip designed to hold a respective chordae;
- the device further comprises a plurality of connecting elements, each connecting element extending between a force sensor and a respective gripping element in order to releasably fix the force sensor to the respective gripping element;
- each connecting element comprises two permanent magnets of opposed polarity designed to cooperate, a first permanent magnet being fixed to the force sensor and a second permanent magnet being fixed to the gripping element associated to the force sensor;
- the device further comprises a processing unit for processing and/or displaying the signals measured by the force sensors;
- for each force sensor, the processing unit is adapted to detect a minimum in the forces measured by the force sensor during its translational displacement with regard to the plate;
- the processing unit is adapted to alert the practitioner when the length of the chordae is near from an optimal length;
- the processing unit is adapted to emit a visual or auditory stimulus when the length of the chordae is near from an optimal length.

### BRIEF DESCRIPTION OF THE DRAWINGS

The present invention may be more completely understood in consideration of the following detailed description of various embodiments in connection with the accompanying drawings, in which:
- Figure 1 is a schematic perspective view of the device for adjusting the length of a neochordae,
- Figure 2 schematic top view of the device for adjusting the length of a neochordae,
- Figure 3 is a schematic view of the device of figure 1 when used on a patient.

### DETAILLED DESCRIPTION OF THE INVENTION

Different examples of the device according to the invention will now be described with reference to the figures. In these different figures, equivalent elements are designated by the same numerical reference.

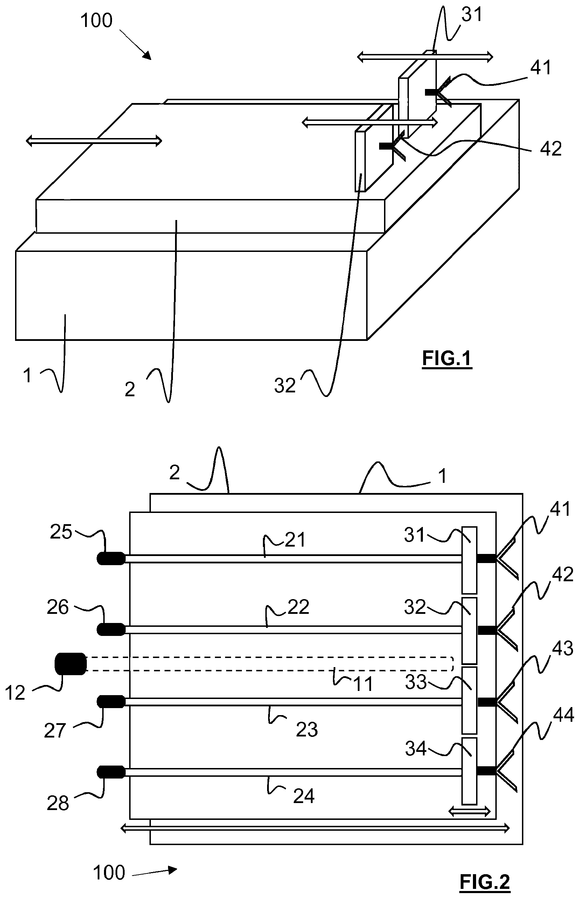

As depicted in figures 1 to 3, the device 100 comprises a base 1 supporting a plate 2 slidably mounted on the base 1, and a plurality of force sensors 31-34 (two force sensors on figure 1, four force sensors on figure 2) slidably mounted on the plate 2, each force sensor 31-34 including a gripping element 41-44 designed for handling a respective neochordae 61-64. Such a device is lightweight, compact, and easy to transport.

### 1. Base

The base 1 can consist in a rectangular metallic platform, such as a linear table.

The base 1 may be fixed on a support foot including a pedestal 81, a stem 82 extending vertically from the pedestal 81, and a carrier 83 fixed on an end of the stem 82 opposite to the pedestal 81, said base 1 being disposed on the carrier 83.

This allows positioning the device 100 near the patient 7 to be treated, at a height sufficient to facilitate the use of the device 100 by the practitioner.

Advantageously, the base 1 comprises primary translational motion element on which the plate 2 is fixed. This allows moving the plate 2 in translation relative to the base 1 in order to modify the tension of all the neochordae 61-64 handled by the plurality of gripping elements 41-44 at the same time. In particular, such a configuration enables the device to apply the same movement - and as a result the same mechanical traction - to all four neochordae 61-64.

In the embodiment illustrated on figure 2, the primary translational motion element comprises a primary endless screw assembly 11 on which the plate 2 is fixed. The primary endless screw assembly 11 is housed in a channel arranged in the base 1. The primary translational motion element further comprises a primary setting wheel 12 fixed on one end of the primary endless screw assembly 11 extending longitudinally outward from the base 1.

The primary endless screw assembly 11 allows translating a rotational motion applied by the practitioner on the primary setting wheel 12 into a linear motion of the plate 2. Preferably, the primary endless screw assembly 11 has a linear travel of 100 millimeters and a pitch distance per revolution of 1 millimeter. This allows a precise synchronous translational displacement of the neochordae 61-64.

Alternatively, the primary translational motion element can consist in a motor embedded in the base 1, said motor being driven by the practitioner using a control panel (not shown).

### 2. Plate

The plate 2 can consist in a rectangular metallic platform - such as a linear table - fixed on the primary translational motion element embedded in the base 1.

Advantageously, the plate 2 comprises a plurality of secondary translational motion elements, each secondary translational motion element supporting a respective force sensor 31-34. Each secondary translation motion element allows the displacement of its associated force sensor 31-34 with regard to the plate 2 in order to individually modify the tension of the neochordae 61-64 handled by the gripping element 41-44 fixed to the force sensor 31-34.

In the embodiment illustrated on figure 2, each secondary translational motion element comprises a secondary endless screw assembly 21-24 on which a respective force sensor 31-34 is fixed. Each secondary endless screw assembly 21-24 is housed in a respective channel arranged in the plate 2. Each secondary translational motion element further comprises a secondary setting wheel 25-28 fixed on one end of the secondary endless screw assembly 21-24 extending longitudinally outward from the plate 2.

Each secondary endless screw assembly 21-24 allows translating a rotational motion applied by the practitioner on the corresponding secondary setting wheel 25-28 into a linear motion of its associated force sensor 31-34. In particular, it allows to independently adjust the transverse tension exerted on each neochordae 61-64 connected to the device 100. Preferably, each secondary endless screw assembly has a linear travel of 100 millimeters and a pitch distance per revolution of 1 millimeter. This allows a precise individual translational displacement of each neochordae 61-64.

Alternatively, each translational motion element can consist in a respective motor embedded in the plate 2, said motors being driven individually by the practitioner using a control panel (not shown).

### 3. Force sensor

Each force sensor 31-34 allows measuring a signal representative of the tension of the neochordae 61-64 hold by its associated gripping element 41-44. More particularly for each neochordae 61 (respectively 62-64), its associated force sensor 31 (respectively 32-34) is adapted to measure the force exerted by the mitral valve on the neochordae 61 (respectively 62-64). This force is representative of the tension of the neochordae 61 (respectively 62-64).

The force range of each force sensor 31-34 can be comprised between -10N and +10N. Moreover the resolution of each force sensor 31-34 may be of 0.01 N. Indeed such force range and resolution (i.e. ± 10 N and 0.01 N respectively) correspond to the constraints usually applied to the neochordae during their implantation.

Each force sensor 31-34 can be of any type known from the skilled person. It comprises electric cables to be connected to an electric power source for powering the force sensor, and (wired or wireless) communication means for transmitting the measured signals to a processing unit (described below).

### 4. Gripping element

Each gripping element 41-44 can consist in a spring clip designed to hold a respective neochordae.

Each spring clip includes:
- two clamp elements pivotably mounted relative to each other, the clamp elements being composed of a first portion for handling the neochordae and of a second portion including tabs for pivotally moving the clamp elements relative to one another,
- a spring member bearing against each of the two clamp elements at an actuating zone thereof, to pivot the two clamp elements to a position closing the first portion.

Advantageously, each gripping element is connected to its associated force sensor with a pivoting link for an ease of handling and comfortable neochordae locking procedure.

Preferably, the device comprises a connecting element associated to each gripping element for releasably mounting the gripping element to its associated force sensor. This allows removing the gripping element from the force sensor in case of accidental movement of the device (for instance if a person bumps into the device during the implantation of the neochordae) in order to limit the risk of damaging the mitral valve (by pulling on the sutures fixed on the valve leaflet).

The connecting element can comprise two permanent magnets of opposed polarity designed to cooperate, a first permanent magnet being fixed to the force sensor and a second permanent magnet being fixed to the gripping element.

### 5. Processing unit

The device can further comprise a processing unit for processing (and/or displaying) the signals measured by the force sensors, and/or for controlling the moving:
- of the plate 2 relative to the base 1, and/or
- of each force sensor relative to the plate 2.

The processing unit can be integrated to the base or may be distant from the base.

The processing unit can consist of one (or several) work station(s) and/or one (or several) computer(s) or may be of any other type known to one skilled in the art. The control unit 6 may for example comprise a portable telephone, an electronic tablet (such as an IPAD®), a Personal Digital Assistant (or "PDA"), etc.

In every case, the processing unit comprises a processor programmed in order to:
- control the sliding of the plate relative to the base (for instance by activating a plate motor associated to the plate), and/or
- control the sliding of each force sensor relative to the plate (for instance by activating sensor motors associated to the respective force sensors), and/or
- process the signals measured by the force sensor, and/or
- display the processed signals on a display element such as a display screen.

With regard to the processing of the signals, the processing unit can be implemented in order to alert the practitioner when the length of the neochordae is nearly the optimal length. In particular for each force sensor, the processing unit can be adapted to detect a minimum in the forces measured by the force sensor during the translational displacement of the force sensor by the practitioner (using the secondary setting wheel).

### 6. Operating Principle

The operating principle of the above described device is the following.

After the implantation - using *Neochord DS 1000* - of one two three or four neochordae by capturing the free edge of the prolapsed leaflet with the capture assembly, the practitioner uses the device according to the present invention in order to adjust the length of each neochordae.

For each neochordae, the practitioner presses the tabs of a gripping element in order to open the first portion of the clamp element. The practitioner places the neochordae into the opened first portion and stop applying a force on the tabs. The two clamp elements pivot due to the force applied by the spring member so that the first portion moves into the closed position for handling the neochordae.

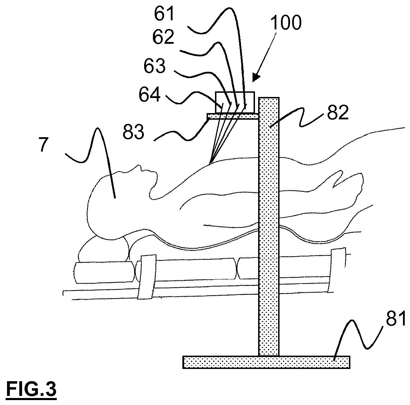

The practitioner repeats the previous steps for the different neochordae sutured to the leaflet. As shown on figure 3, one advantage of the device lies in the fact that it does not come into direct contact with the patient, thereby minimizing the risk of contamination.

Then the practitioner adjusts the length of the all neochordae one after the other.

For each neochordae, the practitioner increases or decreases the length of the neochordae by rotating the secondary setting wheel 25 associated to the neochordae 61. In particular, the rotation of secondary setting wheel 25 induces the translational displacement of the force sensor and gripping element handling the neochordae.

During its displacement, the force sensor continuously measures the signal representative of the tension applied to the neochordae. The processing unit displays the signal measured by the force sensor on the display means in order to facilitate the adjustment of the neochordae's length by the practitioner.

Once the practitioner has defined the optimal length for one neochordae, he repeats the above steps for the other neochordae.

After this step all neochordae are set in tension. An adjustment on all neochordae together can be achieved by rotating the screw 12 under echocardiography control and thanks to the number of neochordae tension.

When a perfect correction is obtained, the neochordae are cut next to the measuring bench and a knot is archived at the right length on the external surface of the heart.

Then the practitioner acts on the primary setting wheel in order to translate the plate with regard to the base. When the length of all neochordae have been adjusted, the practitioner fixes the other ends of the neochordae to the heart.

### 7. Experimental Data

### 7.1. Protocol

The above disclosed device was tested on seven patients with severe mitral regurgitation (MR). All patients had a severe symptomatic MR due to a posterior prolapses, involving mainly P2 segment, with a variable extension on P1 or P3, but never involving the commissures or the anterior leaflet. The procedural success (defined as residual MR < Grade 2) was obtained in six patients.

For all patients, the tension on neochordae was measured throughout the implantation procedure.

The measurement method consists of six steps:
i) Each chord was tested separately to decide which was the most important (generally the neochordae fixed in the middle of the prolapse);
ii) All of the neochordae were then released;
iii) The main neochordae was tracked alone until the best TEE control;
iv) The other neochordae were then tracked, one at a time, with an individual screw to achieve a homogeneous tension on all neochordae;
v) All neochordae were then tracked together using the principal screw under TEE control, until obtaining the optimal MVr with the highest coaptation of the leaflets, and the lowest tension on the neochordae; and
vi) Measurements were then stopped, and the standard protocol was followed.

During all steps, the neochordae tension, EKG, radial blood pressure, 3D TOE, and surgical data were continuously recorded.

### 7.2. Results

*Chordae adjustment, one at a time (Step iii):* While the quality of the control on the correction of the regurgitation was varying from a quasi-perfect MVr to a partial correction, the forces applied on each neochordae always had the same profile. The amplitude of forces increased progressively until a plateau value between 0.7 and 0.9 N (Figure 3). This result was achieved for all patients. Interestingly, in all evaluations, we noticed a variation in the amplitude of tension of approximately 13 % following respiration (Figure 3).

*All neochordae adjustment (Step iv):* When we were setting the tension of the neochordae, one at a time, aiming to achieve the same tension in all neochordae, we observed a decrease in tension on neochordae previously set in tension. At the end of Step iv, the tension initially measured on the first neochordae was divided by the number of neochordae set in tension. Thus, when a tension of 0.8 N was observed with one neochordae, it decreased to 0.2 to 0.3 N when four neochordae are set in tension (see table below).

The mean of the sum of the amplitude of tension in all patients was 0.98 ± 0.08 N.

| **Sequence** | 1 Neochordae | 2 Neochordae | 3 Neochordae | 4 Neochordae |
|---|---|---|---|---|
| Force amplitude Cord1 (N) | 0.79 | 0.41 | 0.35 | 0.21 |
| Force amplitude Cord2 (N) | - | 0.39 | 0.32 | 0.30 |
| Force amplitude Cord3 (N) | - | - | 0.30 | 0.22 |
| Force amplitude Cord4 (N) | - | - | | 0.20 |

*Final adjustment under TEE control (Step v)*: At this point of our protocol, some degree of regurgitation was usually persisting, thus requiring a final adjustment by a synchronous traction on all of the neochordae, until optimal TEE results were achieved. While the initial mean of the sum of the amplitude of tension was 0.98 ± 0.08 N before optimal correction of MR, it decreased gradually to 0.87 ± .07 N following the correction of regurgitation, corresponding to a 12 ± 2 % decrease (range: 10.3-14.1 %) during the correction. When the traction was pursued to the excessive tension, we created a tethering effect with a recurrent Type-III MR. Therefore, we had to release the tension to return to the best MR control situation.

### 7.3. Discussion

Compared to classical MVr, where the neochordae length adjustment on arrested hearts relies mainly on the experience of the practitioner, during novel beating heart techniques, direct, real-time evaluations are made of the quality of the MVr.

This advance has forced practitioner to rethink the approach to MVr and represents a considerable advantage over traditional surgical techniques. With direct TEE control, it is possible to fix the length of the neochordae exactly to the optimal position; neither too long nor too short.

The classical surgical Gore-Tex implantation mostly leads to an overcorrection, with a frozen posterior leaflet, and this likely accounts for why the ring is so important during surgical procedures. This situation might be different for percutaneous techniques, although further studies are required to determine this. Nevertheless, we expect that these types of techniques will grow in popularity as devices and the quality of the 3D images improve. Also, the correction of regurgitation using beating heart techniques represents an opportunity to explore the physiopathology of the mitral valve at every step of the procedure (e.g., clip, rings, chords).

The transapical neochordae implantations allow direct measures of the tension applied on the neochordae of a mitral valve in a human beating heart. These data, formerly inaccessible, are innovative if we consider that data concerning the tensions exerted on the mitral valve are rare.

To date, our knowledge about neochordae tension has relied wholly on in vitro models. Miniature C-shaped force transducers have been most widely used. In this model, the valvular tissue is harvested en masse (ring, valve, and mitral sub-valvular apparatus) from an ovine. The valve and the papillary muscle are attached to a left ventricular simulator, which can mimic the physiological hemodynamic regimen of the left ventricle. A miniature transducer is sutured in the middle of a neochordae.

Interesting data were thus obtained, in particular concerning the differences in tension between marginal, struts, and basal neochordae. The data we obtain are consistent with those found using the miniature C-shaped force transducer model.

Here we report data relevant to the function of the mitral subvalvular apparatus during heart beating. Our tension measurements, recorded throughout the cardiac cycle (from prolapse to tethered chordae), lead us to the following conclusions:
a) The tension applied to a single human mitral chordae is relatively low.
   Our team has already reported the various functions of primary and secondary chordae, and it is important to distinguish their specific roles. The measures presented here are only valid for primary human chordae of 0.7 to 0.8 mm, and which are attached to the free edge of the leaflets. This is probably different for secondary chordae (basal chordae or strut chordae), which are thicker (1.2 to 1.3 mm) and are not involved in neochordae implantations. Dystrophic disease does not lead to secondary chordae rupture so that they are never replaced during MVr. The stress-strain curves of a native chordae have been reported by Zuo, showing ultimate stress values around 35 MPa (equals to 35 N mm-2). In other words, a rupture of chordae should happen if a traction is applied on a normal chordae of 1 mm² and of a prolapsed area if the tension is 35-times superior to the physiologic traction. This likely never happens, meaning that the ruptures of the chordae we encountered among our patients are likely a consequence of the fragility of the diseased chordae, rather than to excessive forces. Gore-Tex® sutures (CV-4 or CV-5) are typically used as neochordae. The diameter of these sutures vary from 0.15 to 0.25 mm and their strength is perfectly adapted since the rupture point is at around 35 N, giving a comfortable safety margin. Moreover, we noticed that the forces applied to the subvalvular apparatus vary with respiration. This might account for why some exceptional thoracic traumatism with the sudden increase of the intra-thoracic pressure can lead to traumatic rupture of normal chordae and acute MR.
b) The tension is divided by the number of chordae set in tension.
   Tension decreased as the number of chordae set in tension increased. This finding, although intuitively expected, is physically confirmed here, with an almost strictly mathematical correlation. The spider's web-structure of the subvalvular apparatus, with numerous chordae and numerous implantation sites on the leaflets, is essential. Intuitively, we could presume that it is also necessary to use several chordae to fix a large prolapse, all along its free edge. Also, an increased number of chordae would likely make the repair less fragile and more durable.
c) The global tension decreases when the control of the regurgitation is optimal
   The normal valve seems to work in a low-stress regimen. In other words, in a physiological state, the perfect systolic closure of the mitral valve with two coapted leaflets inside a 3D saddle shape annular plan corresponds to the situation with the lowest tension on the subvalvular apparatus. Few physiological explanations can be suggested to explain this finding:
   - As mention above, the total tension is divided by the number of chordae. After MR correction, the global tension is distributed between native and artificial chordae. Thus, the tension applied on neochordae (the only one that we can measure) is decreased.

### 8. Conclusion

The above disclosed device allows adjusting the length of neochordae during beating-heart-MVr according to a strict control of their tensions.

In addition, the above described device has allowed the inventor to report original, clinically useful data concerning the physiopathology of the sub-valvular apparatus. Their principal findings are that:
- the tension applied on one human mitral chordae is low;
- the tension is divided by the number of neochordae set in tension; and
- the lowest tension on all neochordae is observed when the control of the regurgitation is optimal.

The scope of the invention is defined by the appended claims.

## Claims

1. Device (100) for assisting a practitioner in adjusting the length of artificial chordae (61-64) implanted in an heart valve, the device (100) comprising a plurality of gripping elements (41-44), each gripping element (41-44) handling an end of a respective artificial chordae (61-64) extending outward from the patient (7) so that the device (100) remains outside from the patient (7),
**characterized in that**
the device (100) further comprises a plurality of force sensors (31-34), each force sensor being fixed to one of the plurality of gripping elements (41-44) for measuring a signal representative of a tension of the chordae (61-64) held by the respective gripping element (41-44) associated to the force sensor.

2. Device according to claim 1, further comprising a plate (2) including a plurality of secondary translational motion elements, each secondary translational motion element supporting a respective force sensor (31-34) so that each force sensor (31-34) is slidably mounted on the plate (2).

3. Device according to claim 2, wherein each secondary translational motion element allows individually moving a respective force sensor (31-34) in translation, each secondary translational motion element including:
- a secondary endless screw assembly (21-24) on which a respective force sensor (31-34) is fixed,
- a secondary setting wheel (25-28) fixed on one end of the secondary endless screw assembly (21-24) extending longitudinally outward from the plate (2).

4. Device according to claim 3, wherein each secondary endless screw assembly (21-24) has a linear travel of 100 millimeters and a pitch distance per revolution of 1 millimeter.

5. Device according to anyone of claims 1 to 4, further comprising a base (1) including a primary translational motion element connected to the plurality of force sensors (31-34) so that each force sensor (31-34) is slidably mounted on the base (1).

6. Device according to claim 5, wherein the primary translational motion element allows synchronously moving the plurality of force sensors (31-34) in translation, the primary translational motion element including:
- a primary endless screw assembly (11) connected to the plurality of force sensors (31-34),
- a primary setting wheel (12) fixed on one end of the primary endless screw assembly (11) extending longitudinally outward from the base (1).

7. Device according to claim 6, wherein the primary endless screw assembly (11) has a linear travel of 100 millimeters and a pitch distance per revolution of 1 millimeter

8. Device according to claims 2 and 5 taken in combination, wherein the plate (2) is fixed on the primary translational motion element so that the plate (2) is slidably mounted on the base (1).

9. Device according to anyone of claims 1 to 8, wherein each gripping element (41-44) consists in a spring clip designed to hold a respective chordae (61-64).

10. Device according to anyone of claims 1 to 9, further comprising a plurality of connecting elements, each connecting element extending between a force sensor (31-34) and a respective gripping element (41-44) in order to releasably fix the force sensor (31-34) to the respective gripping element (41-44).

11. Device according to claim 10,
wherein each connecting element comprises two permanent magnets of opposed polarity designed to cooperate, a first permanent magnet being fixed to the force sensor (31-34) and a second permanent magnet being fixed to the gripping element (41-44) associated to the force sensor (31-34).

12. Device according to anyone of claims 1 to 11, further comprising a processing unit for processing and/or displaying the signals measured by the force sensors (31-34).

13. Device according to claim 12, wherein for each force sensor (31-34), the processing unit is adapted to detect a minimum in the forces measured by the force sensor (31-34) during its translational displacement with regard to the plate (2).

14. Device according to claim 13, wherein the processing unit is adapted to alert the practitioner when the length of the chordae is near from an optimal length.

15. Device according to claim 14, wherein the processing unit is adapted to emit a visual or auditory stimulus when the length of the chordae is near from an optimal length.

## Patentansprüche

1. Vorrichtung (100) zum Unterstützen eines Praktizierenden beim Anpassen der Länge von künstlichen Strängen (61 bis 64), die in einer Herzklappe implantiert sind, wobei die Vorrichtung (100) eine Vielzahl von Greifelementen (41 bis 44) umfasst, wobei jedes Greifelement (41 bis 44) ein Ende eines jeweiligen künstlichen Strangs (61 bis 64), das sich vom Patienten (7) nach außen erstreckt, handhabt, derart, dass die Vorrichtung (100) außerhalb des Patienten (7) bleibt,
**dadurch gekennzeichnet, dass**
die Vorrichtung (100) ferner eine Vielzahl von Kraftsensoren (31 bis 34) umfasst, wobei jeder Kraftsensor zum Messen eines Signals, das für eine Spannung der Stränge (61 bis 64) repräsentativ ist, die vom jeweiligen Greifelement (41 bis 44) gehalten werden, das mit dem Kraftsensor verknüpft ist, an einem der Vielzahl von Greifelementen (41 bis 44) befestigt ist.

2. Vorrichtung nach Anspruch 1, die ferner eine Platte (2) umfasst, die eine Vielzahl von sekundären Translationsbewegungselementen beinhaltet, wobei jedes sekundäre Translationsbewegungselement einen jeweiligen Kraftsensor (31 bis 34) stützt, derart, dass jeder Kraftsensor (31 bis 34) gleitbar auf der Platte (2) montiert ist.

3. Vorrichtung nach Anspruch 2, wobei jedes sekundäre Translationsbewegungselement ein individuelles Bewegen eines jeweiligen Kraftsensors (31 bis 34) in Translation erlaubt, wobei jedes sekundäre Translationsbewegungselement Folgendes beinhaltet:
- eine sekundäre Endlosschraubenanordnung (21 bis 24), an der ein jeweiliger Kraftsensor (31 bis 34) befestigt ist,
- ein sekundäres Einstellrad (25 bis 28), das an einem Ende der sekundären Endlosschraubenanordnung (21 bis 24) befestigt ist und sich in Längsrichtung von der Platte (2) nach außen erstreckt.

4. Vorrichtung nach Anspruch 3, wobei jede sekundäre Endlosschraubenanordnung (21 bis 24) einen linearen Verfahrweg von 100 Millimeter und einen Steigungsabstand pro Umdrehung von 1 Millimeter aufweist.

5. Vorrichtung nach einem der Ansprüche 1 bis 4, die ferner eine Basis (1) umfasst, die ein primäres Translationsbewegungselement beinhaltet, das mit der Vielzahl von Kraftsensoren (31 bis 34) verbunden ist, derart, dass jeder Kraftsensor (31 bis 34) gleitbar auf der Basis (1) montiert ist.

6. Vorrichtung nach Anspruch 5, wobei das primäre Translationsbewegungselement ein synchrones Bewegen der Vielzahl von Kraftsensoren (31 bis 34) in Translation erlaubt, wobei das primäre Translationsbewegungselement Folgendes beinhaltet:
- eine primäre Endlosschraubenanordnung (11), die mit der Vielzahl von Kraftsensoren (31 bis 34) verbunden ist,
- ein primäres Einstellrad (12), das an einem Ende der primären Endlosschraubenanordnung (11) befestigt ist und sich in Längsrichtung von der Basis (1) nach außen erstreckt.

7. Vorrichtung nach Anspruch 6, wobei die primäre Endlosschraubenanordnung (11) einen linearen Verfahrweg von 100 Millimeter und einen Steigungsabstand pro Umdrehung von 1 Millimeter aufweist.

8. Vorrichtung nach den kombinierten Ansprüchen 2 und 5, wobei die Platte (2) derart am primären Translationsbewegungselement befestigt ist, dass die Platte (2) gleitbar an der Basis (1) montiert ist.

9. Vorrichtung nach einem der Ansprüche 1 bis 8, wobei jedes Greifelement (41 bis 44) aus einem Federclip besteht, der zum Halten eines jeweiligen Strangs (61 bis 64) konzipiert ist.

10. Vorrichtung nach einem der Ansprüche 1 bis 9, die ferner eine Vielzahl von Verbindungselementen umfasst, wobei sich jedes Verbindungselement zwischen einem Kraftsensor (31 bis 34) und einem jeweiligen Greifelement (41 bis 44) erstreckt, um den Kraftsensor (31 bis 34) lösbar am jeweiligen Greifelement (41 bis 44) zu befestigen.

11. Vorrichtung nach Anspruch 10, wobei jedes Verbindungselement zwei Permanentmagnete von entgegengesetzter Polarität umfasst, die zum Zusammenwirken konzipiert sind, wobei ein erster Permanentmagnet am Kraftsensor (31 bis 34) befestigt ist und ein zweiter Permanentmagnet am Greifelement (41 bis 44), das mit dem Kraftsensor (31 bis 34) verknüpft ist, befestigt ist.

12. Vorrichtung nach einem der Ansprüche 1 bis 11, die ferner eine Verarbeitungseinheit zum Verarbeiten und/oder zum Anzeigen der Signale, die von den Kraftsensoren (31 bis 34) gemessen werden, umfasst.

13. Vorrichtung nach Anspruch 12, wobei die Verarbeitungseinheit für jeden Kraftsensor (31 bis 34) angepasst ist, bei den Kräften, die vom Kraftsensor (31 bis 34) während seines Translationsversatzes mit Bezug auf die Platte (2) gemessen werden, ein Minimum zu detektieren.

14. Vorrichtung nach Anspruch 13, wobei die Verarbeitungseinheit angepasst ist, den Praktizierenden darauf hinzuweisen, wenn sich die Länge der Stränge in der Nähe einer optimalen Länge befindet.

15. Vorrichtung nach Anspruch 14, wobei die Verarbeitungseinheit angepasst ist, einen visuellen oder akustischen Stimulus zu emittieren, wenn sich die Länge der Stränge in der Nähe einer optimalen Länge befindet.

## Revendications

1. Dispositif (100) pour assister un praticien dans le réglage de la longueur d'un cordage artificiel (61 à 64) implanté dans une valve cardiaque, le dispositif (100) comprenant une pluralité d'éléments de préhension (41 à 44), chaque élément de préhension (41 à 44) manipulant une extrémité d'un cordage artificiel respectif (61 à 64) s'étendant vers l'extérieur à partir du patient (7) de sorte que le dispositif (100) reste à l'extérieur du patient (7),
**caractérisé en ce que**
le dispositif (100) comprend en outre une pluralité de capteurs de force (31 à 34), chaque capteur de force étant fixé à l'un de la pluralité d'éléments de préhension (41 à 44) pour mesurer un signal représentatif d'une tension du cordage (61 à 64) tenu par l'élément de préhension respectif (41 à 44) associé au capteur de force.

2. Dispositif selon la revendication 1, comprenant en outre une plaque (2) incluant une pluralité d'éléments de mouvement de translation secondaires, chaque élément de mouvement de translation secondaire portant un capteur de force respectif (31 à 34) de sorte que chaque capteur de force (31 à 34) est monté de manière coulissante sur la plaque (2).

3. Dispositif selon la revendication 2, dans lequel chaque élément de mouvement de translation secondaire permet de déplacer individuellement un capteur de force respectif (31 à 34) en translation, chaque élément de mouvement de translation secondaire incluant :
- un ensemble de vis sans fin secondaire (21 à 24) sur lequel un capteur de force respectif (31 à 34) est fixé,
- une roue de paramétrage secondaire (25 à 28) fixée sur une extrémité de l'ensemble de vis sans fin secondaire (21 à 24) s'étendant longitudinalement vers l'extérieur à partir de la plaque (2).

4. Dispositif selon la revendication 3, dans lequel chaque ensemble de vis sans fin secondaire (21 à 24) possède une course linéaire de 100 millimètres et une distance de pas par révolution de 1 millimètre.

5. Dispositif selon l'une quelconque des revendications 1 à 4, comprenant en outre une base (1) incluant un élément de mouvement de translation primaire relié à la pluralité de capteurs de force (31 à 34) de sorte que chaque capteur de force (31 à 34) est monté de manière coulissante sur la base (1).

6. Dispositif selon la revendication 5, dans lequel l'élément de mouvement de translation primaire permet de déplacer de manière synchrone la pluralité de capteurs de force (31 à 34) en translation, l'élément de mouvement de translation primaire incluant :
- un ensemble de vis sans fin primaire (11) relié à la pluralité de capteurs de force (31 à 34),
- une roue de paramétrage primaire (12) fixée sur une extrémité de l'ensemble de vis sans fin primaire (11) s'étendant longitudinalement vers l'extérieur à partir de la base (1).

7. Dispositif selon la revendication 6, dans lequel l'ensemble de vis sans fin primaire (11) possède une course linéaire de 100 millimètres et une distance de pas par révolution de 1 millimètre.

8. Dispositif selon les revendications 2 et 5 prises en combinaison, dans lequel la plaque (2) est fixée sur l'élément de mouvement de translation primaire de sorte que la plaque (2) est montée de manière coulissante sur la base (1).

9. Dispositif selon l'une quelconque des revendications 1 à 8, dans lequel chaque élément de préhension (41 à 44) est constitué d'une pince à ressort conçue pour tenir un cordage respectif (61 à 64).

10. Dispositif selon l'une quelconque des revendications 1 à 9, comprenant en outre une pluralité d'éléments de liaison, chaque élément de liaison s'étendant entre un capteur de force (31 à 34) et un élément de préhension respectif (41 à 44) afin de fixer de manière amovible le capteur de force (31 à 34) à l'élément de préhension respectif (41 à 44).

11. Dispositif selon la revendication 10, dans lequel chaque élément de liaison comprend deux aimants permanents de polarité opposée conçus pour coopérer, un premier aimant permanent étant fixé au capteur de force (31 à 34) et un second aimant permanent étant fixé à l'élément de préhension (41 à 44) associé au capteur de force (31 à 34).

12. Dispositif selon l'une quelconque des revendications 1 à 11, comprenant en outre une unité de traitement pour traiter et/ou afficher les signaux mesurés par les capteurs de force (31 à 34) .

13. Dispositif selon la revendication 12, dans lequel pour chaque capteur de force (31 à 34), l'unité de traitement est apte à détecter un minimum parmi les forces mesurées par le capteur de force (31 à 34) au cours de son déplacement en translation par rapport à la plaque (2).

14. Dispositif selon la revendication 13, dans lequel l'unité de traitement est apte à alerter le praticien lorsque la longueur du cordage est proche d'une longueur optimale.

15. Dispositif selon la revendication 14, dans lequel l'unité de traitement est apte à émettre un stimulus visuel ou auditif lorsque la longueur du cordage est proche d'une longueur optimale.
